# EUROPEAN PATENT SPECIFICATION

(11) **EP 1 893 253 B1**
(45) Date of publication and mention of the grant of the patent: **19.05.2010**
(21) Application number: 06727718.6
(22) Date of filing: 23.03.2006
(51) Int. Cl.: A61M 1/02, A61M 1/36

(54) **INTEGRATED SYSTEM FOR COLLECTING, PROCESSING AND TRANSPLANTING CELL SUBSETS, INCLUDING ADULT STEM CELLS, FOR REGENERATIVE MEDICINE**
INTEGRIERTES SYSTEM ZUM SAMMELN, BEARBEITEN UND TRANSPLANTIEREN VON ZELL-SUBSETS, EINSCHLIESSLICH ADULTER STAMMZELLEN, FÜR DIE REGENERATIVE MEDIZIN
SYSTEME INTEGRE PERMETTANT DE RECUEILLIR, TRAITER ET TRANSPLANTER DES SOUS-ENSEMBLES DE CELLULES, Y COMPRIS DES CELLULES SOUCHES ADULTES, POUR LA MEDECINE REGENERATIVE

(30) Priority: 23.03.2005 WO PCT/IB2005/000747
(43) Date of publication of application: 05.03.2008
(73) Proprietor: Biosafe S.A., 1262 Eysins (CH)
(72) Inventor: FELL, Claude, CH-1260 Nyon (CH)
(74) Representative: Cronin, Brian Harold John
(86) International application number: PCT/IB2006/050895
(87) International publication number: WO 2006/100651

(56) References cited:
- EP-A- 1 452 194
- EP-B- 0 912 250
- WO-A-00/38762
- WO-A-03/009889
- US-B1- 6 264 619
- US-B1- 6 655 655

## Description

### FIELD OF THE INVENTION

This invention relates to the collection, automated processing and transplantation of cell subsets as found in the bone marrow, peripheral blood, umbilical cord blood or adipose tissue with the objective to locally reinject these cells for repairing tissues. Cell subsets are typically Adult Stem Cells or platelets but more generally include any sub-populations of cells like red blood cells and white blood cells. Such procedures are likely to be performed in a hospital setting or medical facilities having no cell processing laboratory, and that are likely going to be performed by non-specialized technicians. The invention also describes a new type of optical sensor to monitor cell subsets passing through a transparent tube.

### BACKGROUND OF THE INVENTION

Stem cells are defined as cells that have clonogenic and self-renewing capabilities and that differentiate into multiple cell lineages. Whereas embryonic stem cells are derived from mammalian embryos in the blastocyst stage and have the ability to generate any terminally differentiated cell in the body, adult stem cells are part of tissue-specific cells of the postnatal organism into which they are committed to differentiate. Adult stem cells offer practical advantages over embryonic stem cells. Unlike the latter, they do not raise any ethical issue, and can be extracted from the patient himself. They are in abundant supply and are intrinsic to various tissues of the human body. The most accessible sources of adult stem cells are the bone marrow, peripheral blood, umbilical cord blood and possibly adipose tissues, as indicated by recent studies. These cells are capable of maintaining, generating and replacing terminally differentiated cells within their own specific tissue as a consequence of physiologic cell turnover or tissue damage due to injury. Such capability, known as cell plasticity, has led to the development of therapeutic applications targeting the regeneration of defected tissues, with the goal to restore the physiology and functionality of the affected organ. Adult stem cells can give rise to hematopoiteic cells as known since many decades, but as found in recent years can also give rise to blood vessels, muscles, bone, cartilage, skin, neurons etc. Such cells are known as mesenchymal stem cells. In addition, platelets prepared as platelets concentrate can be used to accelerate wound healing, and consequently can play a role in regenerative medicine to help in the reconstruction of tissues like bone, skin or other tissues.

Hematopoiteic stem cells have been used largely for transplanting patients having undergone chemotherapy in order to restore their hematopoiese. Initially extracted from the bone marrow, they have been sourced more recently from the peripheral blood or umbilical blood, these latter having the highest proliferation capacity. Cells for transplantation require special processing like cell separation, followed sometimes by selection and/or expansion processes. To date, such manipulations have been performed within well-equipped cell-processing laboratories by highly trained personnel that are competent in cell biology and hematology. Such manipulations require labor intensive laboratory preparations involving centrifugation in tubes, density gradient separation, often performed in an open system with the risk of contamination by bacteria, etc.

The new perspectives offered by stem cells in the field of regenerative medicine are challenged by practical issues of manipulating these cells in environments that are unfamiliar with these techniques. One of the main issues is the lack of clean rooms allowing a safe processing of the cells. One can cite as examples the fields of cardiology, orthopedics and neurology that are all experimenting stem cell based therapy, but however lack proper cell processing faculties. Consequently there is a need for simple systems that can process adult stem cells or generally any cell subsets automatically, in a closed system and rapidly in order to provide an on-line cell processing system at the patient's bedside.

### DISCLOSURE OF THE INVENTION

The invention provides a system allowing the extraction, collection, processing and transplantation of cell subsets targeting tissue repair in regenerative medicine. The system according to the invention, as set out in claim 1, comprises a set of disposable sterile elements contained in a pack, the contained elements being pre-connected or including aseptic connectors or being adapted for making interconnections between them in an aseptic manner to provide a functionally closed system. Such system can be offered on a support like a tray that includes individual kits for performing the procedure. The individual kits can be pre-connected or can be equipped with aseptic connectors for making interconnections between them in an aseptic manner, or can be connected using a sterile connecting device like, the SCD from Terumo, operating by welding.

The invention provides a simple system for automatically processing/concentrating cell subsets in a closed system that can provide an on-line cell processing system at the patient's bedside. Embodiments of the invention are set out in the dependent claims.

In one embodiment, the collection container used for harvesting the cell subsets from the patient can be designed in such a way in order to be used as separation chamber as well. Similarly, the receptacle used for collecting the separated cells can be designed in such a way in order to serve as a reinfusion container to deliver the cells back to the patient. The separation of the cells can target a buffy-coat collection or be performed using a density gradient based separation process, followed by a cell washing, based on the system as described in EP-B-912 250 (Claude Fell) and PCT/IB99/020523 (Biosafe). Another way of processing the cells is to use microbeads coated with monoclonal antibodies as described in WO03/009889 (CellGenix/Biosafe).

The combined use of an optical detector which can measure absorption and reflection due to the cells flowing into a transparent tube permits to collect more precisely a particular cell subset like platelets to produce a platelets concentrate. Such platelet concentrate can be obtained in a separate procedure or as a by-product during a procedure targeting a cell subset. The invention also contemplates using the described system for preparing a platelet concentrate for separate use.

The invention thus provides a fully integrated system for bedside intervention that minimizes risks of contamination by using a closed system. It offers a great level of automation and does not rely on any special cell processing expertise. It is suitable for handling any source of cells (such as Adult stem cells, platelets), but particularly for bone marrow stem cell preparation, in an autologous or allogenic setting.

### BRIEF DESCRIPTION OF THE DRAWINGS

The invention will be further described, by way of example, with reference to the accompanying schematic drawings in which:
Fig. 1 is a diagram illustrating the general set-up of a bone marrow processing kit according to the invention;
Fig. 2 shows the symbols used in Figs. 3-7 to illustrate the different components of the illustrated kits according to the invention;
Figs. 3A and 3B show two embodiments of a collection kit, one without and one with a filter unit;
Fig. 4 shows a processing kit that can be connected by an aseptic connector to a collection kit as shown in Fig. 3A or Fig. 3B or to a transplantation kit as shown in Fig. 5A or 5B;
Fig 5A shows individual elements of a transplantation kit and Fig. 5B shows a combination of elements making up a transplantation kit;
Fig. 6 illustrates different combinations of kits for making up a complete system;
Fig. 7 is a diagram of an all-in-one bone marrow processing set in which a rotatable processing chamber constitutes a separation syringe that is used also for collection and transplantation of the cells;
Figs. 7A, 7B and 7C show the operative configurations of components of the set of Fig 7 for collection, processing and transplantation, respectively;
Figs. 8A, 8B and 8C show the principle of the detection of the cells by an optical line sensor using the absorption and reflecting properties of the cells;
Fig 8D shows a vertical view of the optical line sensor with the location of LED and receiver devices; and
Fig. 9 shows typical output signals of the optical line sensor of Fig. 8.

### DETAILED DESCRIPTION

The invention relates to an integrated system allowing the collection of cell subsets, their processing/concentration and reinfusion of a particular cell subset rich product with the objective of repairing or regenerating an injured or defective tissue.

The process will be described in an autologous setting, meaning that the cells are extracted and reinfused from and to the same patient. Such autologous treatment is generally preferred as there is no immunological reaction or adverse effects due to incompatibilities between donor and receivers. However the principle would remain the same in an allogenic setting.

Stem cells, and more specifically mesenchymal stem cells are found in the bone marrow according to current knowledge, although studies indicate that mesenchymal stem cells exist also in umbilical cord blood, peripheral blood or even in fatty tissues. Although the principle would also apply to these various sources of stem cells, the process described here relates to the processing of bone marrow.

The process consists first of a bone marrow extraction from the pelvic zone, under a local anesthesia. Bone is perforated using a bone marrow extractor for example of the Tyco type. The marrow is aspirated using one or multiple syringes, which are pre-filled with some anticoagulant, usually heparin or a citrate/phosphate solution. A volume of 50 ml is typically collected, but could be a different value. The aspirated bone marrow is generally transferred into a PVC collection bag, either filtrated or not, and can be put on an agitator. The PVC collection bag is then connected, using aseptic techniques, preferably on the system described in EP-B-912 250 and PCT/IB99/02052, and separation and concentration of stem cells is then performed accordingly. Other centrifugal processing chambers can be used (e.g. where the rotation axis is not parallel to the axis of a cylindrical processing chamber) or using flexible containers.

EP-B-0 912 250 (C.FELL) describes a system for the processing and separation of biological fluids into components, comprising a set of containers for receiving the biological fluid to be separated and the separated components, and optionally one or more additional containers for additive solutions. A hollow centrifuge processing chamber is rotatable about an axis of rotation by engagement of the processing chamber with a rotary drive unit. The processing chamber has an axial inlet/outlet for biological fluid to be processed and for processed components of the biological fluid. This inlet/outlet leads into a separation space of variable volume wherein the entire centrifugal processing of biological fluid takes place. The processing chamber comprises a generally cylindrical wall extending from an end wall of the processing chamber, this generally cylindrical wall defining therein the hollow processing chamber which occupies a hollow open cylindrical space coaxial with the axis of rotation, the axial inlet/outlet being provided in said end wall coaxial with the generally cylindrical wall to open into the hollow processing chamber. The processing chamber contains within the generally cylindrical wall an axially movable member such as a piston. The separation space of variable volume is defined in an upper part of the processing chamber by the generally cylindrical wall and by the axially movable member contained in the generally cylindrical wall of the processing chamber, wherein axial movement of the movable member varies the volume of the separation space, the movable member being axially movable within the processing chamber to intake a selected quantity of biological fluid to be processed into the separation space via the inlet before or during centrifugal processing and to express processed biological fluid components from the separation space via the outlet during or after centrifugal processing. Means are provided for monitoring the position of the movable member to thereby control the amount of intaken biological fluid and the expression of separated components. The system further comprises a distribution valve arrangement for establishing selective communication between the processing chamber and selected containers or for placing the processing chamber and containers out of communication.

According to PCT/IB99/02052, such a system is arranged to operate in a separation and in a non-separation transfer mode, which provides greater possibilities for use of the system including new applications which were previously not contemplated, such as separation of hematopoietic stem cells and in general laboratory processing. Thus, the system can be arranged to operate such that:
- in the separation mode fluids can be intaken into the processing chamber while the chamber is rotating or stationary, fluid intaken into the chamber is centrifuged and separated into components, and the separated components expressed while the chamber is rotating or, optionally, for the last separated component, while the chamber is stationary; and
- in the transfer mode the processing chamber intakes fluid and expresses fluid with the chamber stationary. The valve actuation arrangement is actuable to transfer amounts of fluid from one container to another via the processing chamber, by moving the member, without centrifugation or separation of the fluid into components, and the means for monitoring the position of the movable member controls the amounts of non-separated fluids transferred.

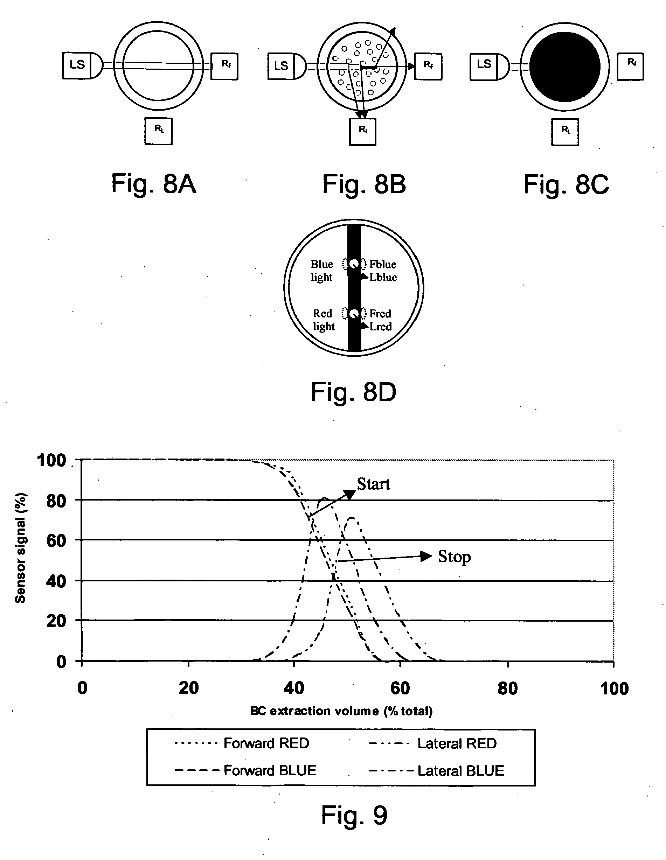

In the new application according to the present invention, which advantageously uses the separation chamber according to EP-B-912 250 and PCT/IB99/02052, separation can target a buffy-coat collection that allows the highest recovery in stem cells without any specific cell subset targeting. The initial product, which sources are those described above, is introduced into the separation chamber by lowering the piston. Once the product has been loaded into the separation chamber - this is detected by the optical line sensor placed near the entry of the separation chamber - a sedimentation cycle of typically 5-10 minutes, produces a buffy-coat layer between the plasma and the red blood cells layer. At the extraction, the plasma is extracted first by moving the piston up. An optical line sensor, for example that described with reference to Fig. 8, detects the cells which belong to the buffy-coat and adapts the different parameters (extraction speed, extracted volume, centrifugation speed) to optimize the cell recovery, depending on the desired volume and the time to process constraints. The buffy-coat cells are extracted to the dedicated bag or vial (depending the configuration of the processing kit). The remaining red blood cells are either extracted to a dedicated bag or kept in the chamber (to save process time). Depending on parameters optimized during the buffy-coat extraction phase, a successive sedimentation / extraction cycle(s) as the one described above can restart. This will complete the buffy-coat extracted volume optimizing its characteristics depending on the final application of the cellular product.

To select a more specific cell subpopulation, the principle described above (filling / sedimentation / extraction) can be used by the same type of optical line sensor which detects by reflection and absorption a more specific cell subset. During the plasma extraction, the absorption and reflection of the liquid are very low. When cells begin to flow in the tube, both absorption and reflection of the effluent product increases. The reflection is also dependant from the type and size of the cells. This dependence permits the selection of a particular cell subset. Finally when the cell concentration is high, the absorption is at the maximum level and reflection is not possible anymore. This can be used to detect cell subsets which have different sizes like platelets and create a platelet concentrate.

To obtain a cell subset selection, a preferred method is to use a density gradient media which targets more specifically a defined cell subpopulation. This will increases the purity of the product, by reducing the contamination in red blood cells and other not wanted cell subsets. The density gradient media is chosen according to the targeted subset. For example, to target mononuclear subset, a Ficoll^{™} based media can be used. In such case, density gradient media is first introduced in the separation chamber. Bone marrow is then slowly introduced by lowering the piston, typically at a rate of 5ml/min, in order to deposit the cells on the layer of density gradient media. Red cells and granulocytes will tend to go through the layer of density gradient media, while mononuclear cells and platelets will remain in front of the layer. When the entire volume of bone marrow is introduced, as detected by an optical line sensor (Fig. 8) on the top of the machine, the piston is stopped and a sedimentation step of, for example, 10-20 min is initiated. An additional dilution could be automatically performed by the system after the complete aspiration of the product, thanks to an isotonic solution connected to the system. Centrifuge speed can be increased to reduce this sedimentation time. Collection then starts by moving the piston up. The liquid supernatant contains only plasma. The first cells then follow, causing the effluent tubing to become opaque, as detected by the optical line sensor. This triggers the collection of the mononuclear cells, which encompass the targeted stem cells. After a volume predefined by the operator menu, or when the effluent line clears again, cell collection stops, and the remaining content of the separation chamber volume is collected in a waste bag until the chamber is completely empty. At this stage, the separation chamber is rinsed from all residual red blood cells thanks to an isotonic solution. The collected cells are reintroduced in the separation chamber, followed, or preceded, by a washing solution like saline / albumine solution (alternatives with phosphate buffered solution or other can be also used). The cells and the washing solution are mixed. The piston will stop after a predetermined volume or when the chamber is completely full. A new sedimentation step is then performed, during which, the collection bag can be washed using the supernatant produced during sedimentation to remove traces of density gradient media. The supernatant (consisting of the washing solution and density gradient media) is then expressed. The process is stopped when the first cells appear again in the effluent line, or can be repeated to obtain a better washing. Cells are finally collected into a collection container that can be specially designed to facilitate further use of the collected cells. When needed the chamber is rinsed. Such cells are readily available for reintroduction into the targeted organ of the patient, or can be further manipulated for selection or expansion purposes. To this effect, the system could re-suspend the cells directly in the desired culture medium.

A more refined separation than using density gradient media consists in incubating the bone marrow in a medium that contains microbeads coated with monoclonal antibodies. Such separation method is described in publication WO03/009889 (CellGenix/Biosafe). The procedure is then as follows. A product containing microbeads linked to a specific antibody is mixed to the blood product containing the cells of interest. After some incubation time, the microbeads will adhere to the surface of the targeted cells, causing their density to change. The mixture is then poured into the separation chamber and a buoyant density separation is initiated as described in earlier patents. When the sedimentation is complete, the supernatant is extracted from the chamber into the waste bag, and then the red cells are also discarded. The cells of interest, marked with the microbeads and therefore having the highest density, will be the last ones to exit the chamber. They can be collected in an appropriate container and, if needed, subsequently washed to remove the antibody solution.

In case of immediate reintroduction after processing has been performed, the collected cells can be connected, using aseptic techniques, to the device allowing their transplantation to the patient. For cardiology applications, such device can be a balloon catheter as used in angiography to locally reinject these cells, like for instance in association with acute myocardial infarction treatments. In this case, a quantity of 10 ml of the concentrated stem cells in steps of 3 ml is reinjected, inflating the balloon at regular interval, allowing the spreading of the stem cells. Such method has been described by Zeiher et al in a scientific paper (TOPCARE - Circulation October 2002).

In case of a platelet concentrate collection, harvested platelets can be used alone or in combination with thrombine possibly obtained as well from the patient's plasma, to form a platelet gel that will facilitate wound healing. Such platelet gel contains growth factors that can advantageously stimulate tissue repair either alone or in association with stem cells.

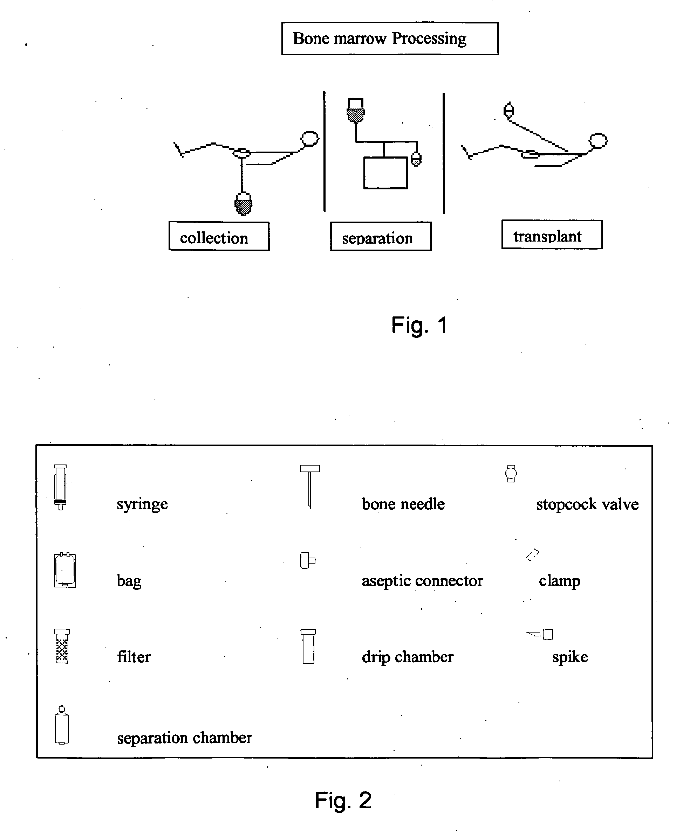

The whole process can be performed at the patient's bedside, and is therefore considered as an on-line process, as illustrated schematically in Fig.1. This provides significant advantages, in safety, logistic and response time, and does not rely on any specific expertise of cell processing.

The collection of various targeted cell subsets can be done during the same collection procedure but with the objective to use such cell subsets at different time intervals during the same operation.

The invention provides a system or "custom pack" that contains already the individual disposable sterile sets for performing the collection, separation and transplantation respectively. Such pack can be presented in a "blister" having 3 compartments each containing a disposable set or kit: one set for the bone marrow extraction, one set for the bone marrow separation, preferably of a type based on the system described in EP-B-912 250 and PCT/IB99/020523 and one set for the reinjection of cells. Each set can have some variations, the one having the highest versatility being the transplantation set, as it depends of the targeted tissue to treat (eg. bone, muscle, vessel, etc). Individual set configurations are illustrated in Figs. 2-7.

Possibly these sets can be preconnected altogether or two of the three can be preconnected, if for instance one wants to use a fully closed system. If not preconnected, a practical solution consists of using specially designed aseptic connectors, like the Medlock system offered by PALL (ref. ACD) and described in US patents 3,650,093, 5,868,433, 6,536,805 and 6,655,655, to ensure that connections are performed under aseptic connections, thus maintaining the criteria of a closed system. Another possibility would be to connect the set using a sterile connecting device. Any of the above configurations - pre-connected, or connected with an aseptic connecting device or a sterile connecting device - will provide a functionally closed system. Such functionally closed systems eliminate the need of clean rooms or laminar flow systems, a very important advantage in operating room or interventional unit environments, which generally are not equipped to meet these requirements.

Another refinement of the invention consists in a bone marrow aspiration container that will act as the separation chamber in second stage, referred as a processing chamber. Its design is similar to the separation chamber as described in PCT/IB99/020523, and it can be fitted with a special needle for perforating the pelvic bone. It is prefilled with anticoagulant or can be primed with anticoagulant prior starting the collection. Once the bone has been perforated, bone marrow is aspirated by moving down the piston of the processing chamber, activated by a manual or electrical vacuum source. The processing chamber is then inserted into the centrifuge of the machine, and a set consisting of an array of tubing lines and bags is connected on the chamber. Separation can then be initiated according to the process described above, using for instance a buffy-coat centrifugation protocol. Another refinement of the invention consists in collecting the separated cells into a special container that can easily be connected or fitted to the system reinfusing the cells back to the patient. Such container can be a graduated syringe fitted with a Y connector having one end connected to the separation set, and the other end equipped with a luer lock connector for subsequent connection to a catheter.

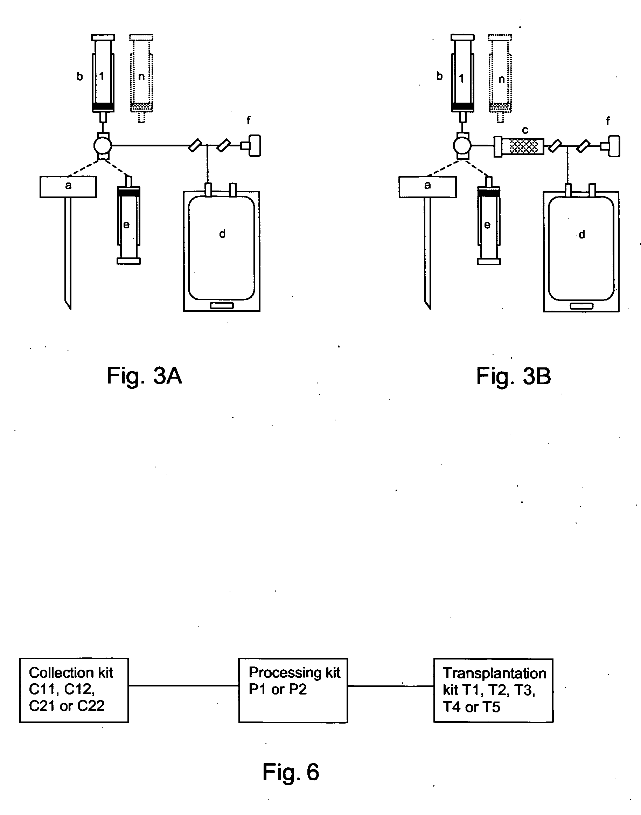

Fig. 3A shows a collection kit without filter and Fig. 3B with filter. The collection kit incorporates everything necessary to perform the marrow aspiration:
a. The input point will for example be a bone marrow extractor (e.g. of the TYCO type) and typically includes a needle for bone puncture or a needle for vein puncture. It can be directly connected to the rest of the kit via a stopcock valve or other valve.
b. One or more syringes (1...n) as needed to perform the aspiration.
c. A filter can be inserted on the collection kit to filter the marrow after collection, as illustrated in Fig. 3B.
d. The collected marrow (filtered or unfiltered) is then stored into a transfer bag prior processing.
e. An additional syringe can be used to add a dilution product to the marrow and/or rinse the filter if applicable.
f. An aseptic connector (Pall ACD or similar) can be used
The collection kit can for example be configured as follows:

| | |
|---|---|
| C11 | Collection kit without filter c with bone needle **a** pre-connected on the processing kit. Quantity X of syringes **b.** |
| C12 | Collection kit without filter with bone needle **a** not pre-connected on the processing kit. Quantity X of syringes **b.** |
| C21 | Collection kit with filter **c** with bone needle **a** pre-connected on the processing kit. Quantity X of syringes **b.** |
| C22 | Collection kit with filter **c** with bone needle **a** not pre-connected on the processing kit. Quantity X of syringes **b.** |

In all these configurations the bone needle can be replaced with a needle for vein puncture.

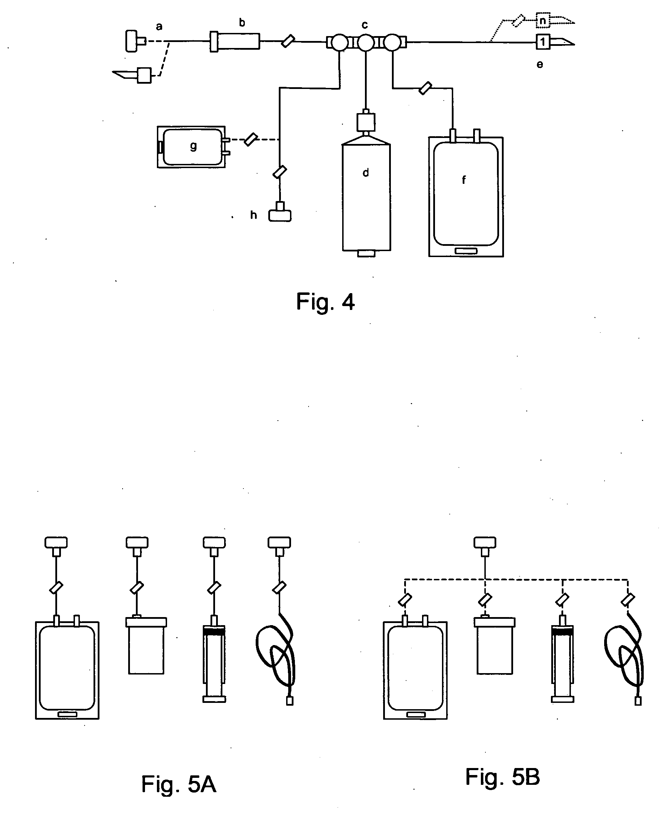

Fig. 4 shows a processing kit that is adapted to be connected to the previously-described collection kit via an aseptic connection.
a. The aseptic connection could be performed via an aseptic connector (such as Pall ACD or others) or a spike connector under aseptic conditions.
b. An optional drip chamber could be used to prevent bubbles entering into the processing path.
c. The direction of the fluid path can be chosen by a stopcock valve ramp mounting three stopcocks in this example, or another type of valve e.g. a multiport valve.
d. A separation chamber for example as described in EP-B-912 250 and PCT/IB99/02052 is used for the separation process.
e. Additional product(s) (isotonic solution, culture medium ,...) is/are entered by a line equipped with one or more connections (spike connectors, ... - 1...n).
f. A satellite or waste bag is used for discarded product and density gradient media input.
g. An output line with an optional intermediate bag guides the product to the transplant kit.
h. An aseptic connector (Pall ACD or similar) could be used on this line.

The main variants of the processing kit include:

| | |
|---|---|
| P1 | processing kit without intermediate bag |
| P2 | processing kit with intermediate bag. |

Fig 5A shows possible individual elements of a transplantation kit and Fig. 5B shows one possible combination of elements making up a transplant kit. The transplantation kit is adapted to be connected to the above described processing kit via an aseptic connection and will contain final product for transplant. The aseptic connection could be performed via an aseptic connector (such as Pall ACD or others) or a spike connector under aseptic conditions.

The transplantation kit could include:

| | |
|---|---|
| T1 | a bag |
| T2 | a collection vial |
| T3 | a syringe |
| T4 | a specific device for transplant (e.g. a catheter for myocardial infarction) |
| T5 | a combination of T1-T4. |

Generally speaking, the transplantation kit will as a minimum include at least one specific device for transplant T4 which can be combined with various combinations of the other components for example a bag T1 or a collection vial T2, and/or a syringe T3.

Fig. 6 illustrates different combinations for making up a complete system. The complete system can for example be composed of any combination of a collection kit (C11 to C22), a processing kit (P1 or P2) and a transplant kit (T1 - T4), as described above.

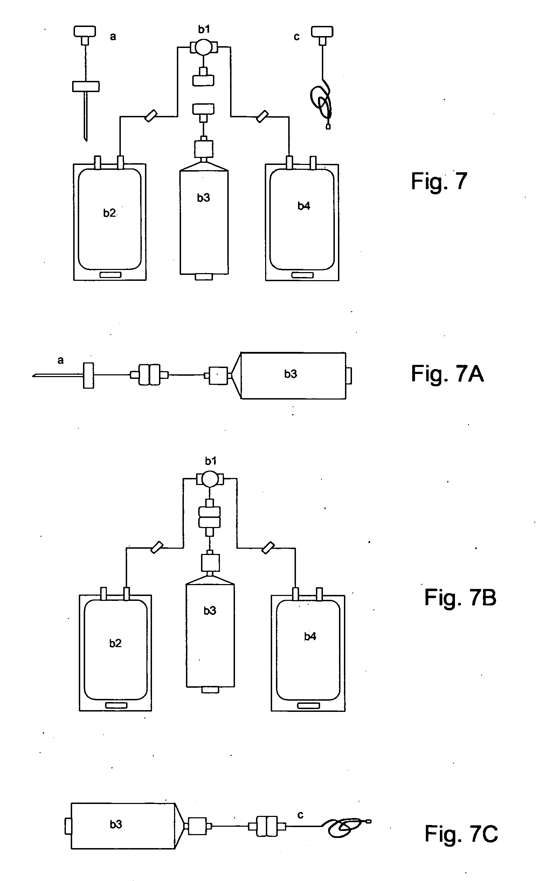

Fig. 7 is a diagram of an all-in-one bone marrow processing set in which a rotatable processing chamber **b3** for example as described in EP-B-912 250 and PCT/IB99/020523 constitutes a separation syringe that is used also for collection and transplantation of the cells. The collection kit **a** consists of the input point, for example a bone marrow extractor (e.g. of the TYCO type) fitted with an aseptic connector for connection to the processing chamber **b3.** The processing kit **b** comprises a stopcock valve **b1** connected to a washing bag **b2** and to a density gradient media/waste bag **b4**, as well as the separating/processing/transplant chamber **b3** that is connectable by an aseptic connector to the stopcock **b1**, or to the collection kit **a,** or to the transplantation kit **c.** The transplantation kit **c** consists of a specific device for transplant (e.g. a catheter for myocardial infarction) fitted with an aseptic connector for connection to the processing chamber **b3.**

Figs. 7A, 7B and 7C show the operative configurations of the components of the set of Fig. 7 for collection, processing and transplantation, respectively.

In Fig. 7A, the collection/processing chamber **b3** is connected by an aseptic connector to the input point of the collection system **a,** so that the processing chamber serves for the collection of the extracted stem cells. Intake of the stem cells is controlled by displacement of the processing chamber **b3**'s piston.

In Fig. 7B, the collection/processing chamber **b3** is connected by its aseptic connector to the stopcock **b1** that connects it selectively to the washing bag **b2** and to the density gradient media/waste bag **b4** for the above-described processing operations, which terminate with the processed/concentrated stem cells being returned to the processing chamber **b3.** Thereafter the processing chamber **b3** serves as reinfusion chamber.

Fig. 7C shows the processing chamber **b3,** after disconnection from the stopcock valve **b1** of the processing kit, connected to the transplant device of the transplantation kit c by an aseptic connector. In this configuration, reinfusion of the processed stem cells into the patient can be controlled by displacement of the processing/reinfusion chamber **b3**'s piston.

This embodiment relies on the use of the aseptic connectors to connect the processing chamber **b3** selectively to the collection kit **a,** or to the remainder of the processing kit via the stopcock valve **b1**, or to the transplantation kit **c** for carrying out the sequential collection, processing and reinfusion operations. This provides a particularly compact system that does not include any non-used elements and is convenient to use.

Figs. 8A, 8B and 8C show the principle of the detection of the cells by an optical line sensor LS using the absorption and reflecting properties of the cells through a transparent tube. Fig. 8A is the configuration of a tube containing clear liquid, where light from LS is unreflected and passes directly to a forward detector R on the light axis. Fig. 8B is the configuration of a tube containing cells in suspension in a clear liquid; in this case the cells reflect light in random directions and is captured both by the forward detector R and by a lateral detector R disposed at about 90° to the axis. Fig. 8C is the configuration of a tube containing opaque liquid where no light is reflected. Fig 8D shows a vertical view of the optical line sensor with the location of LED and receiver devices, in particular showing the positions of the Forward Blue (Fblue), Lateral Blue (Lblue), Forward Red (Fred) and Lateral Red (Lred) light.

Fig. 9 shows the typical signals of the optical line sensor, that are recorded from the "forward" and "lateral" sensors. The information obtained from the "lateral" reflected signals can be used as triggers for starting or ending the collection. The sensor output value (Y axis) is the buffy-coat (BC) extraction volume in percentage of the maximum level. The X axis contains the information of the volume passing through the tube (also in percentage of the total volume).

It is to be understood that this invention may be embodied in several different forms without departing from its spirit or essential characteristics. The scope of the invention is defined in the appended claims, rather than in the specific description preceding them. All embodiments that fall within the meaning of the claims are therefore intended to be embraced by the claims.

## Claims

1. A system for the extraction, collection, processing and transplantation of cell subsets, including adult stem cells and platelets, in particular for organ repair in regenerative medicine, the system comprising a set of disposable sterile fluid-transport elements including:
- an extracting device, for example including a needle for bone or vein puncture, for extracting bone marrow or other sources of cell subsets from a patient;
- at least one chamber for the collection, processing and reinfusion of the cell subsets extracted from the patient, including a collection chamber pre-connected or connectable to the extracting device for harvesting the cells extracted from the patient by the extracting device; a processing chamber adapted to cooperate with processing equipment to perform processing and transfer operations on the harvested cells; and a reinfusion chamber for storing processed cells to be delivered back to the patient; wherein the collection, processing and reinfusion chambers are separate and are pre-connected or interconnectable, or wherein a multi-purpose processing chamber provides the combined functions of a collection-processing chamber, a processing-reinfusion chamber or a collection-processing-reinfusion chamber; and
- a transplantation device pre-connected or connectable to the reinfusion chamber for delivering processed cells back to the patient,
**characterized in that** the set of disposable sterile elements is contained in a pack, the contained elements being pre-connected or including aseptic connectors or being adapted for making interconnections between them in an aseptic manner to provide a functionally closed system.

2. The system of claim 1, wherein the set of disposable elements is packaged in a blister pack on a support such as a tray, the blister pack having one compartment for receiving the entire interconnected set or a plurality of compartments each receiving a part of the set that includes an aseptic connector for connection to another part of the set.

3. The system of claim 1 or 2, wherein the set of disposable elements comprises three kits of disposable elements, a collection kit, a processing kit, and a transplantation kit.

4. The system of claim 3, wherein the collection kit comprises a bone marrow extractor device alone or in combination with: at least one syringe; a transfer bag forming the collection chamber, and optionally an aseptic connector for connection to the processing kit.

5. The system of claim 4, wherein the collection kit further comprises a filter connected or connectable between a syringe and a transfer bag.

6. The system of claim 3, 4 or 5, wherein the processing kit comprises a processing chamber and at least one disposable container connected to the processing chamber via at least one stopcock valve or a multiport valve allowing selective transfer of fluids to and from the processing chamber and to and/or from the disposable container(s), the processing chamber being connected to the collection kit or having an aseptic connector for connection to the collection kit or being adapted for making an aseptic connection, and the processing chamber also being connected to the transplantation kit or having an aseptic connector for connection to the transplantation kit or being adapted for making an aseptic connection.

7. The system of claim 6, wherein the processing kit further comprises a line equipped with one or more connectors for connecting additional containers to the stopcock valve or multiport valve.

8. The system of any one of claims 3 to 8, wherein the transplantation kit comprises at least one transplantation device or a combination of at least one transplantation device with at least one of: a collection bag; a collection vial; and a syringe.

9. The system of any preceding claim, wherein the processing chamber is a hollow centrifugal processing chamber having an inlet/outlet for the cells to be processed and for the processed cells, the processing chamber containing a movable member which defines a separation space of variable size for receiving the cells, the member being movable to intake a selected quantity cells to be processed into the separation chamber via said inlet and to express processed cells from the separation chamber via said outlet.

10. The system of claim 9, wherein the centrifugal processing chamber is generally cylindrical and is rotatable about the cylinder axis, and the movable member is a piston fluid-tightly movably mounted in the centrifugal processing chamber.

11. The system of any preceding claim, which comprises:
- a device for extracting bone marrow or other sources of cell subsets from a patient, said device being connectable by an aseptic connection to the processing chamber for collecting stem cells extracted by the device in the processing chamber;
- at least one disposable container connected to the processing chamber via at least one stopcock valve or a multiport valve allowing selective transfer of fluids to and from the processing chamber and to and/or from the disposable container(s), the processing chamber being connectable to the stopcock or multiport valve via an aseptic connector; and
- at least one transplantation device connectable to the processing chamber by an aseptic connection, for the processing chamber to act as reinfusion chamber for the delivery of processed cells therein to the patient.

12. The system of any preceding claim, wherein the processing chamber is arranged to produce a particular cell subset enriched product (including Adult Stem Cells and platelets).

13. The system of any preceding claim, wherein the processing chamber is arranged to separate the stem cells using a density-gradient based process followed by cell washing.

14. The system of any preceding claim, arranged to process the stem cells using microbeads coated with monoclonal antibodies.

15. The system according to any preceding claim, comprising an optical line sensor for detecting differential reflection of light by a cell subset passing through a transparent tubing.

## Patentansprüche

1. System zur Gewinnung, Sammlung, Verarbeitung und Transplantation von Zell-Subsets, einschließlich adulter Stammzellen und Thrombozyten, insbesondere zur Wiederherstellung von Organen in der regenerativen Medizin, wobei das System ein Set von sterilen Einweg-Flüssigkeitstransportelementen umfasst, das enthält:
- eine Vorrichtung zur Entnahme, die beispielsweise eine Nadel zur Knochen- oder Venenpunktion zur Gewinnung von Knochenmark oder anderen Quellen von Zell-Subsets eines Patienten enthält;
- mindestens eine Kammer zur Sammlung, Verarbeitung und Reinfusion der Zell-Subsets, die von einem Patienten gewonnen werden, einschließlich einer Sammelkammer, die vorkonnektiert oder anschließbar ist an die Entnahmevorrichtung zur Ernte der Zellen, welche vom Patienten durch die Entnahmevorrichtung gewonnen werden; eine Verarbeitungskammer, die mit Zubehör für die Verarbeitung zusammenwirken kann, um die Verarbeitungs- und Transferverfahren der geernteten Zellen durchzuführen; und eine Reinfusionskammer zur Lagerung der verarbeiteten Zellen, welche dem Patienten zurückgegeben werden; wobei die Sammel-, Verarbeitungs- und Reinfusionskammern getrennt sind und vorkonnektiert oder miteinander verbindbar sind, oder wobei eine Mehrzweck-Verarbeitungskammer die kombinierten Funktionen einer Sammel-Verarbeitungskammer, einer Verarbeitungs-Reinfusionskammer oder einer Sammel-Verarbeitungs-Reinfusionskammer bereitstellt; und
- eine Transplantationsvorrichtung, die vorkonnektiert oder anschließbar ist an die Reinfusionskammer zur Rückgabe der verarbeiteten Zellen an den Patienten,
**dadurch** charakterisiert, dass das Set von sterilen Einwegelementen in einer Packung enthalten ist, die enthaltenen Elemente vorkonnektiert sind oder aseptische Anschlüsse enthalten oder geeignet sind zwischen ihnen Verbindungen auf eine aseptische Weise herzustellen, um ein funktionell geschlossenes System bereitzustellen.

2. System nach Anspruch 1, wobei das Set von Einwegelementen in einer Blisterverpackung auf einem Träger, beispielsweise eine Schale, verpackt ist, wobei die Blisterverpackung eine Kammer zur Aufnahme eines vollständigen verbundenen Sets aufweist oder eine Mehrzahl von Kammern aufweist, die jeweils einen Teil des Sets aufnehmen, welcher einen aseptischen Anschluss zur Verbindung mit einem anderen Teils des Sets enthält.

3. System nach Anspruch 1 oder 2, wobei das Set von Einwegelementen drei Kits von Einwegelementen umfasst, einen Sammel-Kit, einen Verarbeitungs-Kit, und einen Transplantations-Kit.

4. System nach Anspruch 3, wobei der Sammel-Kit eine Vorrichtung zur Knochenmarkentnahme allein oder in Kombination umfasst mit: mindestens einer Spritze; einem Transferbeutel, der die Sammel-Kammer bildet, und gegebenenfalls einem aseptischen Anschluss für die Verbindung zum Verarbeitungs-Kit.

5. System nach Anspruch 4, wobei der Sammel-Kit ferner einen Filter umfasst, der zwischen einer Spritze und einem Transferbeutel angeschlossen oder anschließbar ist.

6. System nach Anspruch 3, 4, oder 5, wobei der Verarbeitungs-Kit eine Verarbeitungskammer und mindestens einen Einwegbehälter umfasst, welcher mit der Verarbeitungskammer über mindestens ein Sperrhahn-Ventil oder ein Mehrwegeventil verbunden ist, was eine selektive Übertragung von Flüssigkeiten zu und von der Verarbeitungskammer und zu und/oder vom Einwegbehälter(n) ermöglicht, die Verarbeitungskammer ist mit dem Sammel-Kit verbunden oder weist einen aseptischen Anschluss zur Verbindung zum Sammel-Kit auf oder ist geeignet eine aseptische Verbindung herzustellen, und die Verarbeitungskammer ist ferner verbunden mit dem Transplantations-Kit oder weist einen aseptischen Anschluss zur Verbindung mit dem Transplantations-Kit auf oder ist geeignet eine aseptische Verbindung herzustellen.

7. System nach Anspruch 6, wobei der Verarbeitungs-Kit ferner eine Leitung umfasst, die mit einem oder mehreren Anschlüssen zum Verbinden zusätzlicher Behälter an das Sperrhahn-Ventil oder Mehrwegventil ausgestattet ist.

8. System nach einem der Ansprüche 3 bis 8, wobei der Transplantations-Kit mindestens eine Transplantations-Vorrichtung umfasst oder eine Kombination von mindestens einer Transplantations-Vorrichtung mit zumindest einem von: ein Sammelbeutel; eine Sammel-Ampulle; und eine Spritze.

9. System nach einem der vorangehenden Ansprüche, wobei die Verarbeitungskammer eine hohle Zentrifugationsverarbeitungskammer ist, welche einen Einlass/Auslass für die Zellen, die verarbeitet werden sollen und für die verarbeiteten Zellen aufweist, die Verarbeitungskammer enthält ein bewegbares Element, welches einen Trennraum variabler Größe zur Aufnahme der Zellen definiert, das Element ist bewegbar, um über den Einlass eine ausgewählte Zellmenge aufzunehmen, welche in der Trennkammer verarbeitet werden sollen und um über den Auslass die verarbeiteten Zellen aus der Trennkammer hinauszudrücken.

10. System nach Anspruch 9, wobei die Zentrifugationsverarbeitungskammer üblicherweise zylindrisch ist und um die Zylinderachse drehbar ist, und das bewegbare Element ein Stempel ist, welcher flüssigkeitsdicht beweglich in der Zentrifugationsverarbeitungskammer angeordnet ist.

11. System nach einem der vorangehenden Ansprüche, welches umfasst:
- eine Vorrichtung zur Entnahme von Knochenmark oder anderen Quellen von Zell-Subsets eines Patienten, wobei die Vorrichtung durch eine aseptische Verbindung anschließbar ist an die Verarbeitungskammer zum Sammeln von Stammzellen, die durch die Vorrichtung in der Verarbeitungskammer gewonnen werden;
- mindestens einen Einwegbehälter, der durch mindestens ein Sperrhahn-Ventil oder ein Mehrwegventil mit der Verarbeitungskammer verbunden ist, was eine selektive Übertragung von Flüssigkeiten zu und von der Verarbeitungskammer und zu und/oder vom Einwegbehälter(n) erlaubt, die Verarbeitungsammer ist über einen aseptischen Anschluss mit dem Sperrhahn-Ventil oder Mehrwegventil anschließbar, und
- mindestens eine Transplantations-Vorrichtung, die durch eine aseptische Verbindung an die Verarbeitungskammer anschließbar ist, so dass die Verarbeitungskammer als Reinfusionskammer für die Abgabe von darin verarbeiteten Zellen zum Patienten fungiert.

12. System nach einem der vorangehenden Ansprüche, wobei die Verarbeitungskammer angeordnet ist, um ein bestimmtes angereichertes Zell-Subset-Produkt herzustellen (einschließlich adulte Stammzellen und Thrombozyten).

13. System nach einem der vorangehenden Ansprüche, wobei die Verarbeitungskammer angeordnet ist, um Stammzellen mit Hilfe eines auf einem Dichtegradienten basierenden Verfahrens zu trennen, bei anschließendem Waschen der Zellen.

14. System nach einem der vorangehenden Ansprüche, welches angeordnet ist die Stammzellen mit Hilfe von mit monoklonalen Antikörpern beschichteten Mikrobeads zu verarbeiten.

15. System nach einem der vorangehenden Ansprüche, welches einen optischen Zeilensensor umfasst zur Detektierung unterschiedlicher Lichtreflektion durch ein Zell-Subset, welches durch einen transparenten Schlauch geleitet wird.

## Revendications

1. Système pour l'extraction, la collecte, le traitement et la transplantation des sous-populations de cellules, notamment des cellules souches adultes et des plaquettes, en particulier pour la réparation d'organes en médecine régénérative, le système comprenant un ensemble d'éléments de transport de fluides stériles jetables, comprenant :
- un dispositif d'extraction, par exemple comprenant une aiguille pour ponction osseuse ou veineuse, pour extraire de la moelle osseuse ou d'autres sources de sous-populations de cellules sur un patient ;
- au moins une chambre pour la collecte, le traitement et la reperfusion des sous-populations de cellules extraites sur le patient, notamment une chambre de collecte pré-raccordée ou pré-raccordable au dispositif d'extraction pour récolter les cellules extraites sur le patient par le dispositif d'extraction ; une chambre de traitement adaptée pour coopérer avec un équipement de traitement pour effectuer des opérations de traitement et de transfert sur les cellules récoltées ; et une chambre de reperfusion pour stocker des cellules traitées à redélivrer au patient ; dans lequel les chambres de collecte, de traitement et de reperfusion sont séparées et sont pré-raccordées ou inter-raccordables ou dans lequel une chambre de traitement multiobjectif fournit les fonctions combinées d'une chambre de collecte-traitement, d'une chambre de traitement-reperfusion ou d'une chambre de collecte-traitement-reperfusion ; et
- un dispositif de transplantation pré-raccordée ou raccordable à la chambre de reperfusion pour redélivrer des cellules traitées au patient,
**caractérisé en ce que** l'ensemble d'éléments stériles jetables est contenu dans un conditionnement, les éléments contenus étant pré-raccordés ou comprenant des dispositifs de raccord aseptiques ou étant adaptés pour effectuer des interconnexions entre eux de manière aseptique pour fournir un système fonctionnellement fermé.

2. Système selon la revendication 1, dans lequel l'ensemble d'éléments jetables est conditionné dans un conditionnement alvéolaire sur un support, tel qu'un plateau, le conditionnement alvéolaire ayant un compartiment pour recevoir tout l'ensemble interconnecté ou une pluralité de compartiments recevant chacun une partie de l'ensemble, qui comprend un dispositif de raccord aseptique à raccorder à une autre partie de l'ensemble.

3. Système selon la revendication 1 ou 2, dans lequel l'ensemble des éléments jetables comprend trois trousses d'éléments jetables, une trousse de collecte, une trousse de traitement et une trousse de transplantation.

4. Système selon la revendication 3, dans lequel la trousse de collecte comprend un dispositif de prélèvement de moelle osseuse seul ou en combinaison avec : au moins une seringue ; un sac de transfert formant la chambre de collecte et éventuellement un dispositif de raccord aseptique pour raccorder à la trousse de traitement.

5. Système selon la revendication 4, dans lequel la trousse de collecte comprend en outre un filtre raccordé ou raccordable entre une seringue et un sac de transfert.

6. Système selon la revendication 3, 4 ou 5, dans lequel la trousse de traitement comprend une chambre de traitement et au moins un récipient jetable raccordé à la chambre de traitement via au moins une soupape de robinet d'arrêt ou une soupape multiorifice permettant un transfert sélectif de fluides vers la chambre de traitement ou depuis celle-ci et vers le ou les récipients jetables et/ou depuis ceux-ci, la chambre de traitement étant raccordée à la trousse de collecte ou ayant un dispositif de raccord aseptique pour raccorder à la trousse de collecte ou étant adaptée pour créer un raccord aseptique, et la chambre de traitement étant également raccordée à la trousse de transplantation ou ayant un dispositif de raccord aseptique pour raccorder à la trousse de transplantation ou étant adaptée pour créer un raccord aseptique.

7. Système selon la revendication 6, dans lequel la trousse de traitement comprend en outre un conduit équipé d'un ou plusieurs dispositifs de raccord pour raccorder des récipients additionnels à la soupape du robinet d'arrêt ou à la soupape multiorifice.

8. Système selon l'une quelconque des revendications 3 à 8, dans lequel la trousse de transplantation comprend au moins un dispositif de transplantation ou une combinaison d'au moins un dispositif de transplantation avec au moins au choix un sac de collecte ; un flacon de collecte et une seringue.

9. Système selon l'une quelconque des revendications précédentes, dans lequel la chambre de traitement est une chambre de traitement centrifuge creuse ayant une entrée/sortie pour les cellules à traiter et pour les cellules traitées, la chambre de traitement contenant un élément mobile qui définit un espace de séparation de taille variable pour recevoir les cellules, l'élément étant mobile pour amener une quantité sélectionnée de cellules à traiter dans la chambre de séparation via ladite entrée et pour extraire les cellules traitées de la chambre de séparation par ladite sortie.

10. Système selon la revendication 9, dans lequel la chambre de traitement centrifuge est généralement cylindrique et peut tourner autour de l'axe du cylindre, et l'élément mobile est un piston étanche aux fluides monté mobile dans la chambre de traitement centrifuge.

11. Système selon l'une quelconque des revendications précédentes, qui comprend :
un dispositif pour extraire de la moelle osseuse ou d'autres sources de sous-populations de cellules sur un patient, ledit dispositif pouvant être raccordé par un raccord aseptique à la chambre de traitement pour collecter des cellules souches extraites par le dispositif dans la chambre de traitement ;
au moins un récipient jetable raccordé à la chambre de traitement via au moins une soupape de robinet d'arrêt ou une soupape multiorifice permettant un transfert sélectif de fluides vers la chambre de traitement ou depuis celle-ci et/ou vers le ou les récipients jetables et/ou depuis ceux-ci, la chambre de traitement pouvant être raccordée à la soupape du robinet d'arrêt ou à la soupape multiorifice via un dispositif de raccord aseptique ; et
au moins un dispositif de transplantation raccordable à la chambre de traitement par un raccord aseptique, pour que la chambre de traitement agisse comme chambre de reperfusion pour y délivrer des cellules traitées au patient.

12. Système selon l'une quelconque des revendications précédentes, dans lequel la chambre de traitement est aménagée pour produire un produit particulier enrichi en sous-populations de cellules (notamment des cellules souches adultes et des plaquettes).

13. Système selon l'une quelconque des revendications précédentes, dans lequel la chambre de traitement est aménagée pour séparer les cellules souches en utilisant un procédé basé sur un gradient de densité suivi d'un lavage des cellules.

14. Système selon l'une quelconque des revendications précédentes, aménagé pour traiter les cellules souches en utilisant des microbilles revêtues d'anticorps monoclonaux.

15. Système selon l'une quelconque des revendications précédentes, comprenant un capteur de ligne optique pour détecter une réflexion différentielle de lumière par une sous-population de cellules traversant un tube transparent.
